# EUROPEAN PATENT SPECIFICATION

(11) **EP 0 959 824 B1**
(45) Date of publication and mention of the grant of the patent: **08.05.2002**
(21) Application number: 97935898.3
(22) Date of filing: 14.08.1997
(51) Int. Cl.: A61F 5/01

(54) **ANKLE-FOOT-ORTHESIS**
SPRUNGGELENK-FUSS-ORTHESE
ORTHESE POUR CHEVILLE ET PIED

(30) Priority: 16.08.1996 NL 1003826
(43) Date of publication of application: 01.12.1999
(73) Proprietor: Orteam B.V., 5211 's-Hertogenbosch (NL)
(72) Inventor: VAN DE KAMP, Arnoldus, Hendrikus, Gerardus, NL-5241 XS Rosmalen (NL)
(74) Representative: Ottevangers, Sietse Ulbe
(86) International application number: NL9700464
(87) International publication number: WO9807395

(56) References cited:
- EP-A- 0 466 100
- WO-A-95/33426
- DE-A- 3 228 753
- NL-A- 9 100 420
- US-A- 4 289 122
- US-A- 5 334 135

## Description

The invention relates to an ankle-foot orthesis comprising a thin-walled boot-shaped body, wherein at least a part of the front side of the boot-shaped body is open so that, in use, parts of the shinbone and the instep of the foot are not covered by the boot-shaped body, and at least one fastening strap which is connected to the boot-shaped body and which, in use, together with the boot-shaped body encloses the leg.

Such ankle-foot orthesis is known from EP-A-0 466 100 and can be worn by people having paresis of the foot levators.

During walking, such foot tends to drop around the ankle joint. In the case of a spastic paresis, a foot that is bent downwards relative to the leg starts to go tense and, possibly, move up and down rapidly.

The object of the ankle-foot orthesis is to have the foot assume an angle of about 90° relative to the upper leg. In the case of a paresis, this has as an advantage that a patient having such disorder can walk more or less normally again. When the patient suffers from a spastic paresis, the vibrating of the foot proves to decrease substantially as soon as the foot is forced to assume an angle of about 90° relative to the lower leg.

The advantage of the known ankle-foot orthesis is that it is manufactured from a thin-walled material, so that it is in principle possible to wear the orthesis in combination with normal shoes. The drawback of the known ankle-foot orthesis, however, is that it is rather stiff during walking. As a consequence, the patient cannot properly wind off his foot during walking, as in the case of natural walking.

By 'winding off' during walking is meant in particular that the angle included by the foot and the lower leg can become smaller when, during walking, the weight on the foot is displaced from the heel to the toes. During walking, the ankle joint will then always assume the same position. As a consequence, the patient walks visibly uneasily and, moreover, quickly becomes tired. Moreover, an irksome sliding movement proves to occur between the top part of the orthesis and a part of the leg which is located above the ankle and covered by the ankle-foot orthesis.

If during walking a very substantial force is exerted on the body, to such an effect that the angle enclosed between a sole of the boot-shaped body and a vertical rear wall of the boot-shaped body is decreased, vertical sidewalls of the boot-shaped body will deflect outwards at the level of the ankle. If this happens frequently, the thin-walled boot-shaped body will tear at the level of the ankle.

The object of the invention is to provide a solution to the above-described problems. Accordingly, the ankle-foot orthesis according to the invention is characterized in that in the boot-shaped body, at the level of the ankle, a plurality of slots are provided which are mutually separated from each other in vertical direction and extend in a first vertical sidewall of the boot-shaped body, a vertical rear wall of the boot-shaped body covering at least a part of the rear side of the leg below the knee, and a second vertical sidewall of the boot-shaped body located opposite the first vertical sidewall.

According to the invention, the slots act as a particular joint. The ankle-foot orthesis according to the invention now has the property of being in the first instance stiff. However, when during walking a sufficiently great force is exerted on the body, the above-mentioned angle between the sole of the foot and the vertical rear wall may slightly decrease. This involves the connection between the foot sole and the vertical rear wall being springy. As a result, the sole rebounds into its original position again when the weight is taken from the leg. When this angle between the sole and the vertical rear wall decreases, the width of the slots will increase slightly. However, this does not involve any outward deflection of the sidewalls at the level of the ankles, causing wear in the thin-walled body.

Further, the joint formed by the slots proves to behave like an ankle joint. This means that the pivotal movement of the ankle-foot orthesis does not only involve a change of the angle enclosed between the sole and the vertical rear wall, but also an upward movement of the top portion of the ankle-foot orthesis relative to the sole when this angle becomes smaller. This is precisely the movement that is made by a natural ankle joint. Hence, it concerns a combined motion of rotation and translation. As a result, during walking, no chafing movement is caused between the top portion of the ankle-foot orthesis and the patient's leg.

The slots also involve the possibility of increasing this enclosed angle through a limited angle at the moment when the heel is placed on the ground during a forward movement. During this portion of the movement, the slots are squeezed, as it were. When the slots are squeezed, it is not possible to increase the enclosed angle any further. Hence, the ankle-foot orthesis according to the invention has as a characteristic that it is resiliently bendable, wherein the enclosed angle can decrease by a relatively large angle and can be springily bent, wherein the enclosed angle can decrease at a relatively large angle and can be springily bent in an opposite direction, wherein this angle can increase through a relatively small angle, wherein further bending is not possible, and that the top portion of the ankle-foot orthesis moves upwards relative to the sole when this enclosed angle decreases.

DE-A-32 28 753 discloses a foot-supporting means which is not partially open at the front side, so that in use, the shinbone and the instep of the foot are indeed covered via the boot-shaped body. Hence, this concerns a product different from the present invention. Moreover, slots are provided which extend along the front side rather than along the rear side of the boot-shaped body. The object of these slots is to enable an adaptation of the foot-supporting means, which is designed as a serial product, to the individual foot, while it is moreover achieved that the foot can easily be inserted into the supporting means. Because the slots are provided at the front side, however, they cannot act as a joint, as described in relation to the present invention.

Preferably, in the ankle-foot orthesis according to the invention, each of the slots extends from positions in the first vertical sidewall via the vertical rear wall to positions in the second vertical sidewall. Such ankle-foot orthesis has the advantage that a proper spring action is obtained without the occurrence of wear in the form of tear formation in the material. In this context, the term 'sidewall' should be given a broad interpretation. This means that the rear wall can be defined as being very narrow, so that the vertical sidewalls almost touch each other. However, it is essential that the slots extend along the rear side of the ankle-foot orthesis.

Preferably, the body is provided, at the ends of a number of slots, with a throughbore. The throughbore has as a result that the above-described springy movement can be carried out even more easily without the occurrence of wear in the material of the body of the ankle-foot orthesis, also after many years of use.

In particular, the body comprises a long and a short slot, alternating in vertical direction. When the enclosed angle decreases during walking, the bending in the body of the ankle-foot orthesis will in particular be effected by the long slots. Also when this enclosed angle increases by the above-mentioned limited angular value, this will result from the squeezing of the slots. However, when in particular circumstances a particularly great force is exerted on the ankle-foot orthesis, in such a manner that the enclosed angle threatens to be further increased, the short slots will be squeezed. The enclosed angle can hence further increase by a predetermined limited value through the squeezing of the short slots. When both the long and the short slots are squeezed, further bending of the ankle-foot orthesis in a direction wherein this angle increases is no longer possible. Consequently, the thus obtained ankle-foot orthesis has the characteristic that it can in the first instance be bent through a limited angle, wherein the long slots are squeezed, and can only be bent any further in the case of a strongly increasing force, wherein the short slots are squeezed.

Ankle-foot ortheses utilizing a brace which is attached to the leg and pivotally connected to a sole of the foot are known per se. However, these braces are provided with metal bars and have the drawback that they are difficult to wear in a concealed manner because of their robust dimensions. Moreover, the movement of the joint does not follow the movement of the ankle joint, so that during walking, the top portion of the ankle-foot orthesis will chafe up and down along the leg.

The invention will now be further explained with reference to the accompanying drawings. In these drawings:
Fig. 1a is a side elevational view of an ankle-foot orthesis known per se;
Fig. 1b is a rear view of the ankle-foot orthesis according to Fig. 1a;
Fig. 1c is a cross section of the ankle-foot orthesis according to Figs. 1a, 2a and 3a;
Fig. 2a is a side elevational view of a first embodiment of an ankle-foot orthesis according to the invention;
Fig. 2b is a rear view of the ankle-foot orthesis according to Fig. 1b;
Fig. 3a is a side elevational view of a part of the ankle-foot orthesis according to Fig. 2a in unbent condition;
Fig. 3b is a side elevational view of a part of the ankle-foot orthesis according to Fig. 2a in bent condition;
Fig. 4a is a side elevational view of a second embodiment of an ankle-foot orthesis according to the invention;
Fig. 4b is a rear view of the ankle-foot orthesis according to Fig. 4a;
Fig. 5a is a side elevational view of a part of the ankle-foot orthesis according to Fig. 4a in unbent condition;
Fig. 5b is a side elevational view of a part of the ankle-foot orthesis according to Fig. 4a in a condition in which it is bent in a first direction;
Fig. 5c is a side elevational view of a part of the ankle-foot orthesis according to Fig. 4a in a condition in which it is slightly bent in a second direction;
Fig. 5d is a side elevational view of a part of the ankle-foot orthesis according to Fig. 5a in a condition in which it is bent further in the second direction relative to the condition in Fig. 5c;
Fig. 6a is a side elevational view of a third embodiment of an ankle-foot orthesis according to the invention;
Fig. 6b is a rear view of the ankle-foot orthesis according to Fig. 6a;
Fig. 7a is a side elevational view of a fourth embodiment of an ankle-foot orthesis according to the invention; and
Fig. 7b is a rear view of the ankle-foot orthesis according to Fig. 7a.

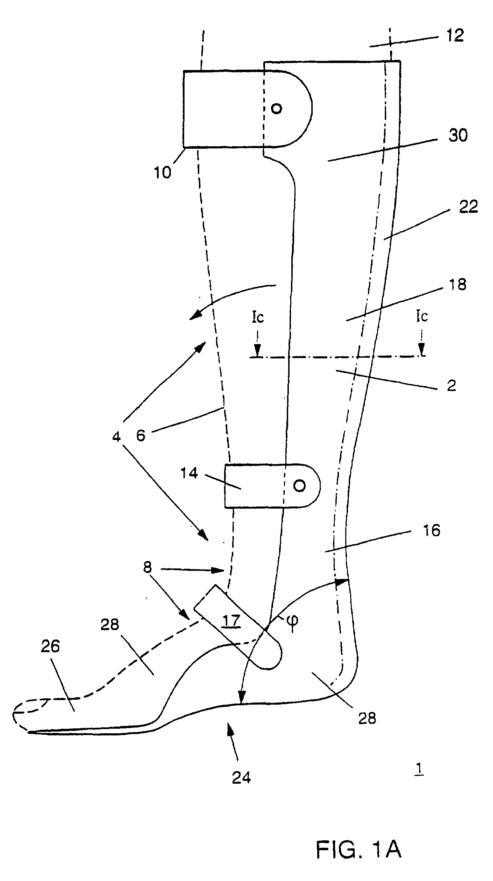

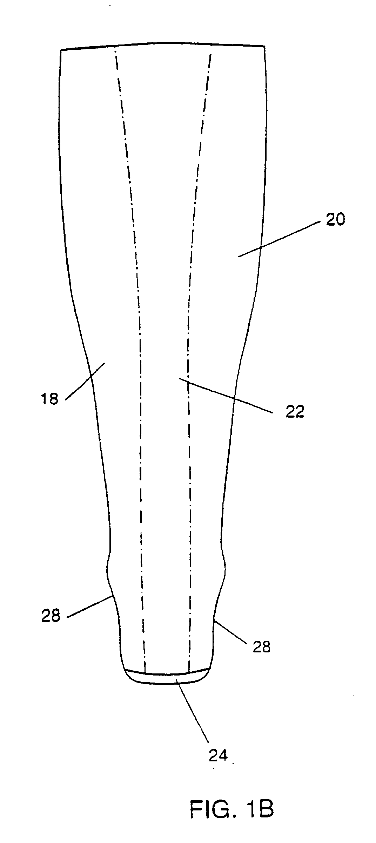

In Figs. 1a and 1b, reference numeral 1 shows an ankle-foot orthesis which is known per se. The ankle-foot orthesis comprises a thin-walled boot-shaped body 2. A part 4 of the body 2 is open, so that, in use, parts of the shinbone 6 and the instep 8 of the foot are not covered by the body. In Fig. 1a, the front side of the shinbone 6 and the top side of the foot are shown in dotted lines.

The ankle-foot orthesis further comprises a first fastening strap 10, which is connected to the boot-shaped body and which, in use, together with the boot-shaped body encloses the leg 12. For this purpose, the fastening strap 10 is detachably connected to the boot-shaped body 2 by means of elements known per se, the arrangement being such that the fastening strap 10 can be shortened, so that the leg is firmly pressed into the boot-shaped body.

In this example, the ankle-foot orthesis further comprises a second fastening strap 14 located at a height above the ankle 16, and a third fastening strap 17, extending over the instep 8 of the foot. The second and third fastening straps, too, are connected to the boot-shaped body 2 so that they can be detached and tightened.

The boot-shaped body 2 is manufactured from a resilient rigid material, such as for instance polypropylene. The boot-shaped body comprises a first vertical sidewall 18 and a second vertical sidewall 20, located opposite the first vertical sidewall. The vertical sidewalls 18, 20 each cover at least a part of the lateral sides of the foot and at least a part of the lateral sides of the leg from the foot up to a height below the knee. As Fig. 1a shows, the vertical sidewalls are in fact of L-shaped design. Further, a portion of the thin-walled body extending at the rear of the leg from the heel upwards can be referred to as a vertical rear wall 22. In Figs. 1a and 1b, the vertical rear wall is shown in dot-dash lines. The boot-shaped body 2 further comprises a sole part 24, extending from the heel to a position below the toes 26. By tightening the fastening straps 10, 14, 17, the leg 12 is pressed into the boot-shaped body. The rear side of the leg will hence be pressed firmly against the inside of the vertical sidewall 22. Further, the bottom side of the foot will be pressed firmly against the inside of the sole part 24. Also, the vertical sidewalls 18, 20 will be pressed firmly against the lateral sides of the foot and the leg. As a result, a foot 28 will assume a fixed angle relative to the shinbone 6, defined by the ankle-foot orthesis. Thus, the foot is prevented from dropping in the case of a flaccid paresis or from going tense and, as a result, quickly moving back and forth in the case of a spasm in the calf.

However, a drawback of the orthesis according to Fig. 1a is that it is entirely stiff. This means that a user cannot wind off his foot during walking. When a very great force is exerted on the boot-shaped body so that the angle ϕ between sole part 24 of the boot-shaped body and the vertical rear wall 22 decreases, opposite ankle parts 28 of the boot-shaped body 2 will be bent outwards. If this happens too often, the rigid material of the body will tear at that location. A further drawback of the known ankle-foot orthesis is that during walking, a top portion 30 of the ankle-foot orthesis will move up and down along the leg 12. This usually causes an irksome situation for the patient.

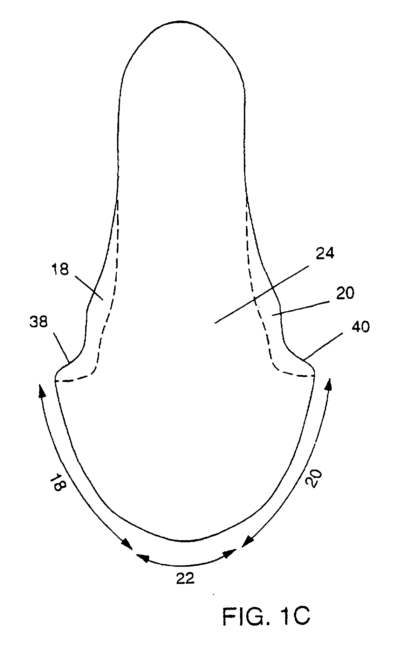

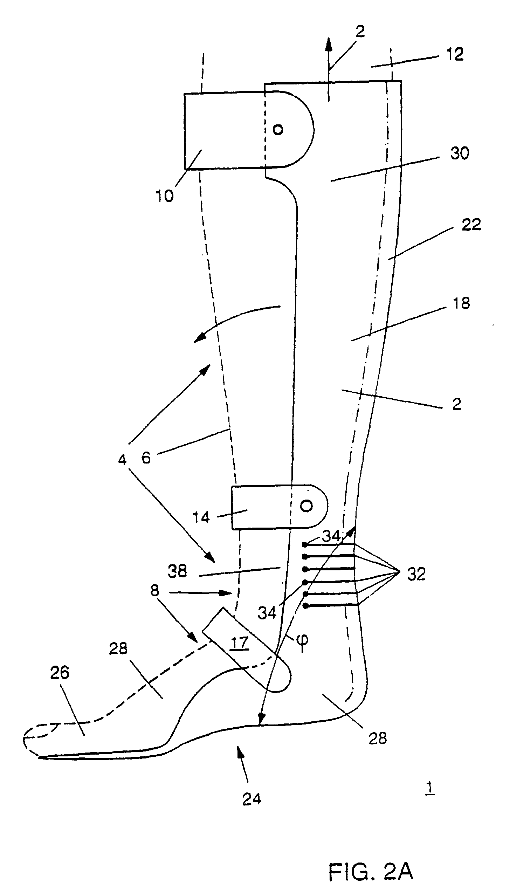

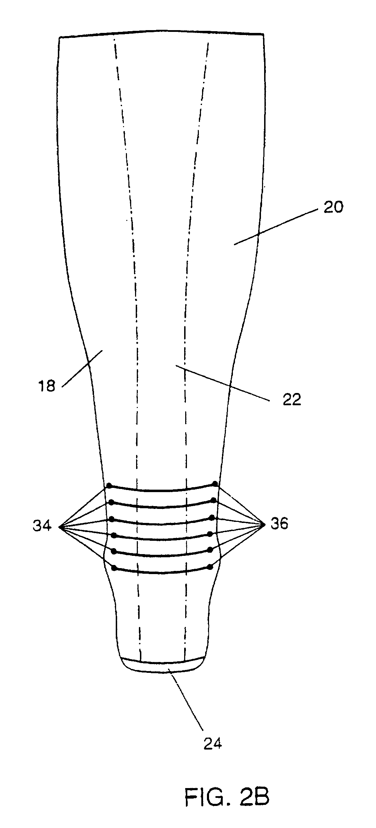

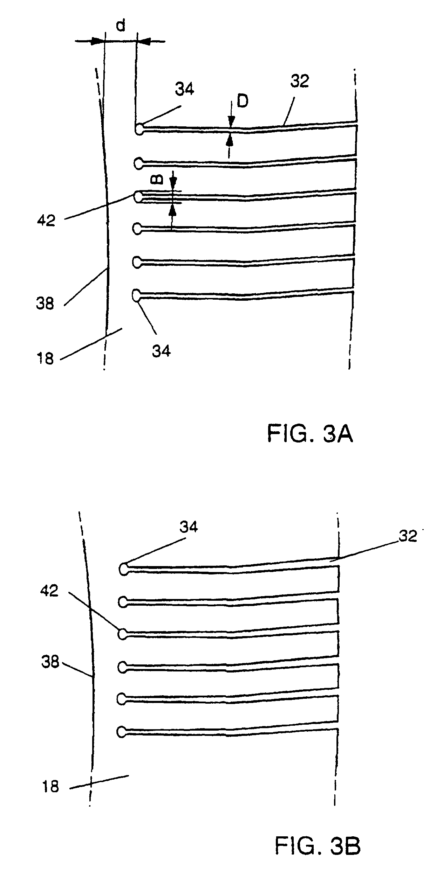

An ankle-foot orthesis which meets the above-mentioned problems is shown in Figs. 2a, 2b, 3a and 3b. Here, parts corresponding to the ankle-foot orthesis according to Figs. 1a, 1b and 1c are provided with identical reference numerals. The boot-shaped body is manufactured from a resilient rigid material such as for instance polypropylene. However, other materials are also possible. In the ankle-foot orthesis according to the invention, the thin-walled boot-shaped body is provided, approximately at the level of the ankle, with a plurality of slots 32 separated from one another in vertical direction. The slots extend from a position 34 in the first vertical sidewall 18 to a position 36 in the second vertical sidewall 22 of the body, located opposite the first vertical sidewall. Each of the slots 32 extends at least substantially arcuately in a plane which is directed at least substantially horizontally, i.e. in a plane which is approximately parallel to the sole part 24. This is not essential, because the plane may also assume other positions. At any rate, the slots will extend at least substantially arcuately in a non-vertically directed plane. Hence, in this example, each of the slots has a first end 34 located in the first vertical sidewall 18, and a second end 36 located in the second vertical sidewall 22. In this example, the first ends 34 are located adjacent a vertical longitudinal edge 38 of the first sidewall 18, and the second ends 36 are located adjacent a vertical longitudinal edge 40 of the second vertical sidewall 20. In this example, the distance d between the first ends, respectively the second ends, and the adjacent vertical longitudinal edges is 1-4 cm. In this example, the body comprises six slots. However, other numbers are also possible, such as for instance four to fifteen slots. In particular, as shown in Figs. 3a and 3b, the body is provided, at the first and second ends of a number of slots and in this case even all slots, with a throughbore 42. The throughbore 42 has a diameter B which is larger than the width D of the slots. In particular, the throughbores have a diameter of 1.5-8 mm, and the width D of the slots is 0.3-1.2 mm.

The operation of the ankle-foot orthesis according to Fig. 2a is as follows. During walking, it is possible at the moment when a user displaces his weight from the heel to the toe 26, that the upper part 30 of the boot-shaped body bends slightly forwards relative to the sole part 24. This will involve a decrease of the angle ϕ. This situation is further explained with reference to Figs. 3a and 3b. In Fig. 3a, the body of the ankle-foot orthesis is in unbent condition. In Fig. 3b, however, the angle ϕ has slightly decreased. As shown in Fig. 3b, the slots are bent open with spring action, as it were. The width D of the slots will therefore increase. Owing to the throughbores 42, a properly springing construction is obtained, while it is ensured that the material of the boot-shaped body at the ends 34, 36 of the slots 32 will not tear. As a result of the spring action of the boot-shaped body, the ankle-foot orthesis will, when the weight is taken from the foot, rebound into its original condition, as shown in Fig. 3a. When the angle ϕ decreases during walking, the upper side 30 of the boot-shaped body will move slightly upwards, in a direction indicated in Fig. 2a by the arrow Z. This is exactly the movement that is also made by the leg 12 when the upper leg is moved forwards relative to the foot by means of bending around the ankle joint. The result is that the slots form a joint which approaches the natural motion of the ankle joint. This prevents a chafing to-and-fro movement being caused between the upper side of the boot-shaped body and the leg 12 during walking.

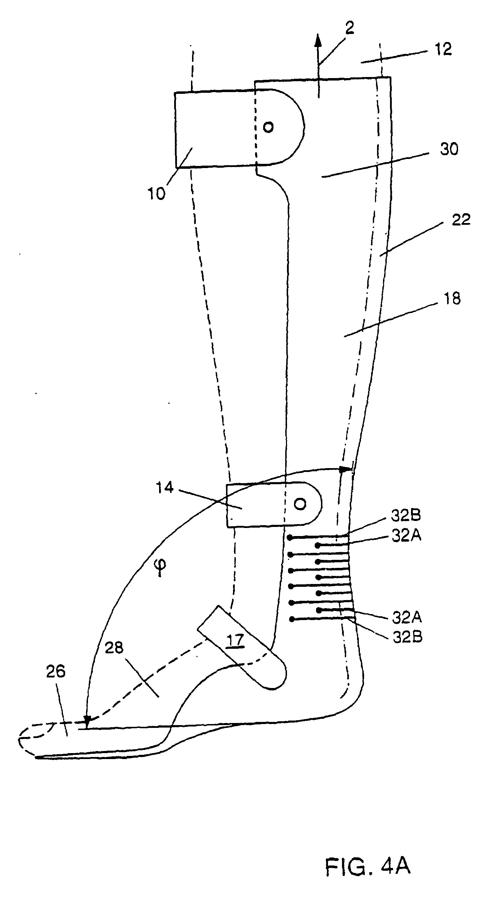

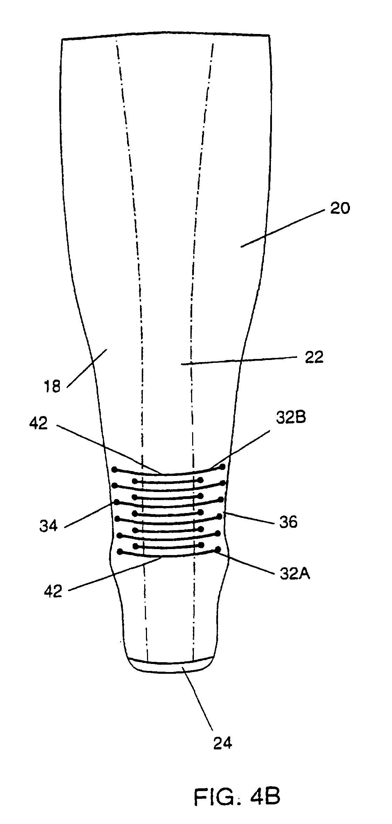

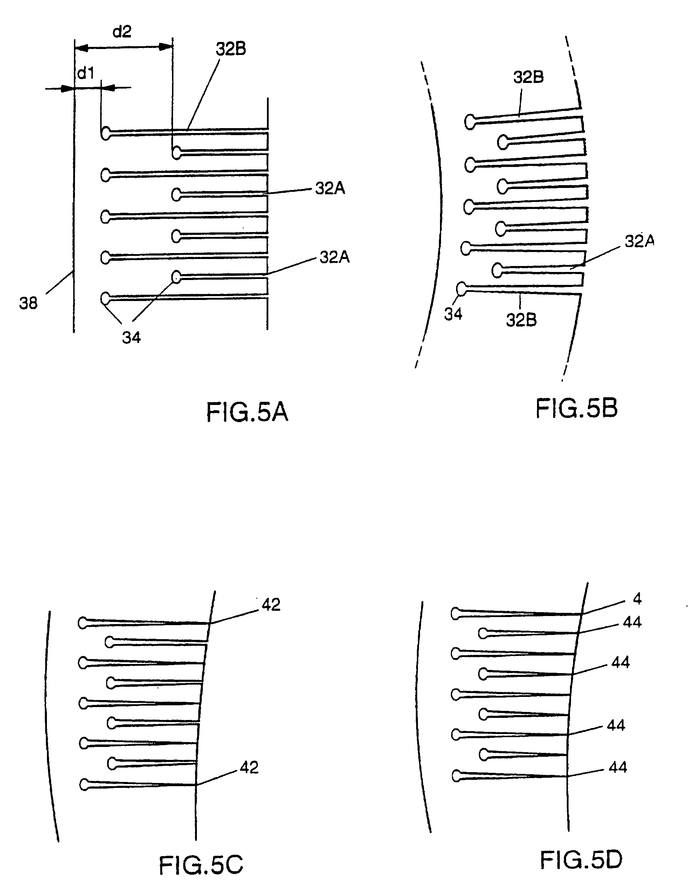

Figs. 4a, 4b and 5a-5c show a second possible embodiment of an ankle-foot orthesis according to the invention. In Figs. 2 and 3 on the one hand and Figs. 4 and 5 on the other, corresponding parts are again provided with identical reference numerals. Here, the boot-shaped body 2 is provided with a number of slots having mutually different lengths. In this example, the boot-shaped body 2 comprises slots which are alternately long and short in vertical direction. The short slots 32a have about the same length relative to each other, and the long slots 32b also have about the same length relative to each other. The distance dl between the vertical longitudinal edge 38 and the ends 34, 36 of the long slots 32b is in this example about 2 cm. The corresponding distance d2 for the short slots 32a is in this example about 6 cm (see also Fig. 5a).

The operation of the ankle-foot orthesis according to Fig. 4 is further explained with reference to Figs. 5a-5d.

When, during walking, the weight of the heel is displaced to the foot, the ankle-foot orthesis will spring in such a manner that the angle ϕ decreases, as discussed in relation to Figs. 3a and 3b. This situation is shown in Fig. 5b. In this situation, substantially the long slots 32b will be bent open. As the distance d2 is larger than d1, the short slots 32a will not be bent open. Hence, the springy characteristic of the ankle-foot orthesis in a direction wherein the angle ϕ decreases is at least substantially determined by the assembly of the long slots 32b. When, during a new step forwards, the weight of the foremost leg is subsequently placed on the heel, it is desired that the angle ϕ can increase through a limited angle. The increase of the angle ϕ should be limited, because the foot must be prevented from folding downwards too far. If this happened to a patient having a spastic paresis, the foot would start vibrating and going tense again. In the first instance, the angle ϕ cannot increase any further than a condition in which the long slots 32b at the rear of the boot-shaped body (in the vertical rear wall 22) are closed. At the locations 42 indicated in Figs. 5c and 4b, the long slots 32b are closed and, consequently, the angle ϕ will not be able to increase any further when the force on the boot in the relevant direction increases only relatively slightly. In the situation of Fig. 5c, the short slots 32a are not yet closed. This is again caused by the fact that the distance d2 is much larger than the distance d1, so that more force is required for closing the short slots 32a than the long slots 32b. However, when, in particular situations, yet greater forces are exerted on the ankle-foot orthesis, in a direction in which they urge the angle ϕ to increase, the short slots 32a can be closed as well, when the force is sufficiently great. The slots are squeezed at locations 44 which are likewise in the vertical rear wall 22. In the situation as shown in Fig. 5d, the angle ϕ has maximally increased. Because now, as shown in Fig. 5d, all slots are closed, it is not possible to increase the angle ϕ any further. The springy characteristics of the ankle-foot orthesis in a direction in which the angle ϕ increases are therefore limited, i.e. the angle ϕ can increase by a predetermined value only, which value is inter alia determined by the number of slots and the width of the slots. Hence, the ankle-foot orthesis according to Fig. 4 has the property that during bending of the ankle-foot orthesis in a direction in which the angle ϕ decreases, the force required therefor is determined by the long slots 32b. On the other hand, bending in an opposite direction, in which the angle ϕ increases, and the associated maximum increase of the angle ϕ, are determined by the assembly of the long and short slots. Hence, both phenomena can be dimensioned independently of each other.

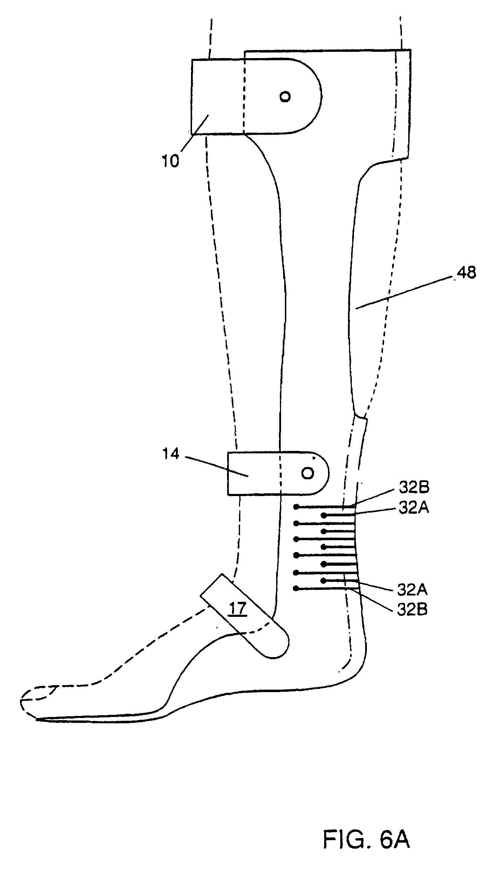

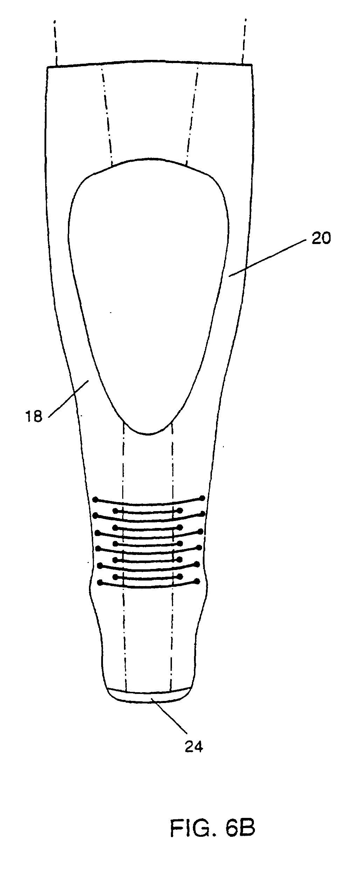

A third possible embodiment of the ankle-foot orthesis according to the invention is shown in Figs. 6a and 6b. The ankle-foot orthesis entirely corresponds to that of Figs. 5a and 5b, the difference being that an opening 48 is provided in the boot-shaped body above the slots 32a and 32b. This opening renders the ankle-foot orthesis lighter and moreover has as a result that a larger portion of the leg is cooled by fresh air.

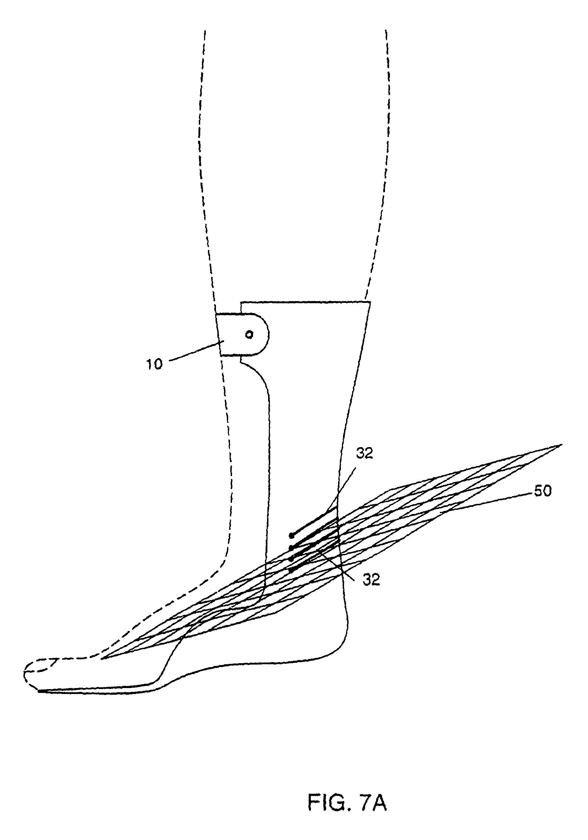

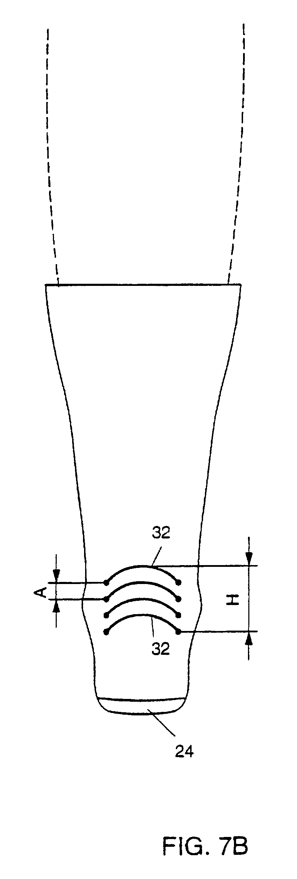

Finally, in Figs. 7a and 7b, a fourth embodiment of an ankle-foot orthesis according to the invention is shown. In these Figures, parts corresponding to earlier Figures have been provided with the same reference numerals. The ankle-foot orthesis according to Figs. 7a and 7b comprises a boot-shaped body which is lower than the boot-shaped body of the embodiments described hereinabove. Moreover, the ankle-foot orthesis comprises only one fastening strap 10. Further, a number of slots 32 have been provided approximately at the level of the ankle. The slots 32 are arcuate and each extend at least substantially in a plane which is not vertically directed. However, in this example, the relevant planes are not horizontally directed either. In Fig. 7a, only the plane 50 is shown, in which the lowest slot 32 extends. The planes in which the other slots extend are approximately parallel to the plane 50 and are located above the plane 50. The operation and use of the ankle-foot orthesis according to Figs. 7a and 7b is otherwise analogous with the operation of the exemplary embodiments described hereinabove.

To most embodiments it applies that the mutual distance A between two adjacent slots is about 4-20 mm (see Fig. 7b). In particular, the mutual distance between adjacent slots of different adjacent slots is approximately equal. This is the case with all embodiments shown. In the above-described embodiments, the slots are provided in a path extending in vertical direction at the level of the ankle through a distance H of about 4-10 cm (see Fig. 7b). However, values other than the values given above are also possible. The invention is by no means limited to the embodiments outlined hereinabove. For instance, toes of the foot may also be covered by the boot-shaped body. Higher parts of the leg may in principle also be covered by the boot-shaped body. The number of slots, the length of the slots, the width of the slots, the position of the slots, and the number of fastening straps may likewise be varied. Also, the material from which the boot-shaped body is manufactured may vary. For this, different types of plastic may be used, while it is moreover not precluded that a thin-walled metal is used. These and other readily occurring variants are all understood to fall within the framework of the invention.

## Claims

1. An ankle-foot orthesis (1) comprising a thin-walled boot-shaped body (2) wherein at least a part (4) of the front side of the boot-shaped body is open so that, in use, parts of the shinbone (6) and the instep (8) of the foot are not covered by the boot-shaped body, and at least one fastening strap (10) which is connected to the boot-shaped body and which, in use, together with the boot-shaped body encloses the leg (12), **characterized in that** in the boot-shaped body (2), at the level of the ankle, a plurality of slots (32) are provided which are mutually separated from each other in vertical direction and extend in a first vertical sidewall (18) of the boot-shaped body, a vertical rear wall of the boot-shaped body covering at least a part of the rear side of the leg below the knee, and a second vertical sidewall (20) of the boot-shaped body located opposite the first vertical sidewall.

2. An ankle-foot orthesis according to claim 1, **characterized in that** the slots extend from positions in the first vertical sidewall, via the vertical rear wall, to positions in the second vertical sidewall.

3. An ankle-foot orthesis according to claim 1 or 2, **characterized in that** the slots extend at least substantially arcuately in a non-vertically directed plane.

4. An ankle-foot orthesis according to claim 3, **characterized in that** the slots extend at least substantially arcuately in a plane which is directed at least substantially horizontally.

5. An ankle-foot orthesis according to any one of the preceding claims, **characterized in that** at least one of the slots has a first end located in the first vertical sidewall and a second end located in the second vertical sidewall.

6. An ankle-foot orthesis according to claim 5, **characterized in that** the first end is located adjacent a vertical longitudinal edge of the first sidewall, and that the second end is located adjacent a vertical longitudinal edge of the second sidewall.

7. An ankle-foot orthesis according to claim 6, **characterized in that** the distance between the first end, respectively the second end, and the adjacent vertical longitudinal edges is 1-9 cm.

8. An ankle-foot orthesis according to any one of the preceding claims, **characterized in that** the body comprises a number of slots having different lengths relative to one another.

9. An ankle-foot orthesis according to claim 8, **characterized in that** the body comprises a long and a short slot, alternating in vertical direction.

10. An ankle-foot orthesis according to claim 9, **characterized in that** the short slots have about the same lengths and that the long slots have about the same lengths.

11. An ankle-foot orthesis according to any one of the preceding claims, **characterized in that** the body comprises 4-15 slots.

12. An ankle-foot orthesis according to any one of the preceding claims, **characterized in that** the mutual distance between two adjacent slots is 4-20 mm.

13. An ankle-foot orthesis according to any one of the preceding claims, **characterized in that** the mutual distance between adjacent slots is approximately equal for different adjacent slots.

14. An ankle-foot orthesis according to any one of the preceding claims, **characterized in that** the width of the slots is 0.3-1.2 mm.

15. An ankle-foot orthesis according to any one of the preceding claims, **characterized in that** the body is provided, at the ends of a number of slots, with a throughbore.

16. An ankle-foot orthesis according to claim 15, **characterized in that** a number of throughbores have a diameter of 1.5-8 mm.

17. An ankle-foot orthesis according to any one of the preceding claims, **characterized in that** the slots are provided in a path extending in vertical direction at the level of the ankle through a distance of about 4-10 cm.

18. An ankle-foot orthesis according to any one of the preceding claims, **characterized in that** the body is provided with an opening located above the slots.

19. An ankle-foot orthesis according to any one of the preceding claims, **characterized in that** a first fastening strap is located at a height above the slots.

20. An ankle-foot orthesis according to claim 18, **characterized in that** a second fastening strap is located at the level of the instep of the foot.

21. An ankle-foot orthesis according to any one of the preceding claims, **characterized in that** the boot-shaped body is manufactured from a resilient rigid material.

## Patentansprüche

1. Knöchel-Fuß-Orthese (1) mit einem dünnwandigen stiefelförmigen Körper (2), bei dem wenigstens ein Teil (4) der Vorderseite des stiefelförmigen Körpers offen ist, so daß im Gebrauch Teile des Schienbeins (6) und des Spanns (8) des Fußes nichtvom stiefelförmigen Körper bedeckt sind, und mit wenigstens einem Befestigungsriemen (10), der mit dem stiefelförmigen Körper verbunden ist, und der im Gebrauch zusammen mit dem stiefelförmigen Körper das Bein (12) umschließt, **dadurch gekennzeichnet, daß** im stiefelförmigen Körper (2) auf der Höhe des Knöchels mehrere Schlitze (32) vorgesehen sind, die voneinander in vertikaler Richtung beabstandet sind und sich in einer ersten vertikalen Seitenwand (18) des stiefelförmigen Körpers, einer vertikalen Rückwand des stiefelförmigen Körpers, die wenigstens einen Teil der Rückseite des Beins unterhalb des Knies bedeckt, und einer zweiten vertikalen Seitenwand (20) des stiefelförmigen Körpers erstrecken, die derersten vertikalen Seitenwand gegenüberliegt.

2. Knöchel-Fuß-Orthese nach Anspruch 1, **dadurch gekennzeichnet, daß** sich die Schlitze von Positionen in der ersten vertikalen Seitenwand über die vertikale Rückwand zu Positionen in der zweiten vertikalen Seitenwand erstrecken.

3. Knöchel-Fuß-Orthese nach Anspruch 1 oder 2, **dadurch gekennzeichnet, daß** sich die Schlitze wenigstens im wesentlichen gebogen in einer nicht vertikal ausgerichteten Ebene erstrecken.

4. Knöchel-Fuß-Orthese nach Anspruch 3, **dadurch gekennzeichnet, daß** sich die Schlitze wenigstens im wesentlichen gebogen in einer Ebene erstrecken, die wenigstens im wesentlichen horizontal gerichtet ist.

5. Knöchel-Fuß-Orthese nach einem der vorhergehenden Ansprüche, **dadurch gekennzeichnet, daß** wenigstens einer der Schlitze ein erstes Ende, das in der ersten vertikalen Seitenwand angeordnet ist, und ein zweites Ende aufweist, das in der zweiten vertikalen Seitenwand angeordnet ist.

6. Knöchel-Fuß-Orthese nach Anspruch 5, **dadurch gekennzeichnet, daß** sich das erste Ende nahe einem vertikalen Längsrand der ersten Seitenwand befindet, und daß sich das zweite Ende nahe einem vertikalen Längsrand der zweiten Seitenwand befindet.

7. Knöchel-Fuß-Orthese nach Anspruch 6, **dadurch gekennzeichnet, daß** der Abstand zwischen dem ersten bzw. dem zweiten Ende und dem benachbarten Längsrand 1-9 cm beträgt.

8. Knöchel-Fuß-Orthese nach einem der vorhergehenden Ansprüche, **dadurch gekennzeichnet, daß** der Körper wenigstens eine Anzahl von Schlitzen mit in bezug zueinander unterschiedlichen Längen aufweist.

9. Knöchel-Fuß-Orthese nach Anspruch 8, **dadurch gekennzeichnet, daß** der Körper einen langen und einen kurzen Schlitz aufweist, die in vertikaler Richtung abwechseln.

10. Knöchel-Fuß-Orthese nach Anspruch 9, **dadurch gekennzeichnet, daß** die kurzen Schlitze ungefähr die selbe Länge haben und daß die langen Schlitze ungefähr die selbe Länge haben.

11. Knöchel-Fuß-Orthese nach einem der vorhergehenden Ansprüche, **dadurch gekennzeichnet, daß** der Körper 4-15 Schlitze aufweist.

12. Knöchel-Fuß-Orthese nach einem der vorhergehenden Ansprüche, **dadurch gekennzeichnet, daß** der gegenseitige Abstand zwischen zwei benachbarten Schlitzen 4-20 mm beträgt.

13. Knöchel-Fuß-Orthese nach einem der vorhergehenden Ansprüche, **dadurch gekennzeichnet, daß** der gegenseitige Abstand zwischen benachbarten Schlitzen für unterschiedliche benachbarte Schlitze ungefähr gleich ist.

14. Knöchel-Fuß-Orthese nach einem der vorhergehenden Ansprüche, **dadurch gekennzeichnet, daß** die Breite der Schlitze 0,3 - 1,2 mm beträgt.

15. Knöchel-Fuß-Orthese nach einem der vorhergehenden Ansprüche, **dadurch gekennzeichnet, daß** der Körper an den Enden einer Anzahl von Schlitzen mit einer Durchgangsbohrung versehen ist.

16. Knöchel-Fuß-Orthese nach Anspruch 15, **dadurch gekennzeichnet, daß** eine Anzahl von Durchgangsbohrungen einen Durchmesser von 1,5 bis 8 mm hat.

17. Knöchel-Fuß-Orthese nach einem der vorhergehenden Ansprüche, **dadurch gekennzeichnet, dass** die Schlitze in einer Bahn vorgesehen sind, die sich in vertikaler Richtung auf der Höhe des Knöchels über einen Abstand von etwa 4-10 cm erstreckt.

18. Knöchel-Fuß-Orthese nach einem der vorhergehenden Ansprüche, **dadurch gekennzeichnet, dass** der Körper mit einer Öffnung versehen ist, die sich oberhalb der Schlitze befindet.

19. Knöchel-Fuß-Orthese nach einem der vorhergehenden Ansprüche, **dadurch gekennzeichnet, dass** ein erster Befestigungsriemen sich auf einer Höhe oberhalb der Schlitze befindet.

20. Knöchel-Fuß-Orthese nach Anspruch 18, **dadurch gekennzeichnet, dass** ein zweiter Befestigungsriemen sich auf der Höhe des Spanns des Fußes befindet.

21. Knöchel-Fuß-Orthese nach einem der vorhergehenden Ansprüche, **dadurch gekennzeichnet, dass** der stiefeiförmige Körper aus einem elastischen steifen Material hergestellt ist.

## Revendications

1. Gouttière (1) pour pied et cheville, comprenant un corps (2) en forme de botte à paroi mince dans laquelle une partie au moins (4) de la face avant du corps en forme de botte est dégagée afin que, pendant l'utilisation, des parties du tibia (6) et du cou-de-pied (8) du pied ne soient pas couvertes par le corps en forme de botte, et au moins une sangle de fixation (10) qui est raccordée au corps en forme de botte et qui, pendant l'utilisation, avec le corps en forme de botte, entoure la jambe (12), **caractérisée en ce que**, dans le corps en forme de botte (2), au niveau de la cheville, plusieurs fentes (32) sont disposées et mutuellement séparées les unes des autres en direction verticale et s'étendent dans une première paroi latérale verticale (18) du corps en forme de botte, dans une paroi verticale arrière du corps en forme de botte recouvrant une partie au moins de la face arrière de la jambe au-dessous du genou, et dans une seconde paroi latérale verticale (20) du corps en forme de botte placée en face de la première paroi latérale verticale.

2. Gouttière pour pied et cheville selon la revendication 1, **caractérisée en ce que** les fentes s'étendent de positions de la première paroi latérale verticale à des positions de la seconde paroi latérale verticale par l'intermédiaire de la paroi verticale arrière.

3. Gouttière pour pied et cheville selon la revendication 1 ou 2, **caractérisée en ce que** les fentes s'étendent au moins pratiquement en courbe dans un plan qui n'est pas vertical.

4. Gouttière pour pied et cheville selon la revendication 3, **caractérisée en ce que** les fentes s'étendent au moins sous forme pratiquement courbe dans un plan qui est au moins pratiquement horizontal.

5. Gouttière pour pied et cheville selon l'une quelconque des revendications précédentes, **caractérisée en ce que** l'une au moins des fentes a une première extrémité disposée dans la première paroi latérale verticale et une seconde extrémité disposée dans la seconde paroi latérale verticale.

6. Gouttière pour pied et cheville selon la revendication 5, **caractérisée en ce que** la première extrémité est adjacente à un bord longitudinal vertical de la première paroi latérale, et la seconde extrémité est adjacente à un bord longitudinal vertical de la seconde paroi latérale.

7. Gouttière pour pied et cheville selon la revendication 6, **caractérisée en ce que** la distance comprise entre la première extrémité, respectivement la seconde extrémité, et les bords longitudinaux verticaux adjacents est comprise entre 1 et 9 cm.

8. Gouttière pour pied et cheville selon l'une quelconque des revendications précédentes, **caractérisée en ce que** le corps comprend un certain nombre de fentes ayant des longueurs différentes les unes des autres.

9. Gouttière pour pied et cheville selon la revendication 8, **caractérisée en ce que** le corps comporte une fente longue et une fente courte qui alternent en direction verticale.

10. Gouttière pour pied et cheville selon la revendication 9, **caractérisée en ce que** les fentes courtes ont à peu près la même longueur, et les fentes longues ont à peu près la même longueur.

11. Gouttière pour pied et cheville selon l'une quelconque des revendications précédentes, **caractérisée en ce que** le corps comporte de quatre à quinze fentes.

12. Gouttière pour pied et cheville selon l'une quelconque des revendications précédentes, **caractérisée en ce que** la distance entre deux fentes adjacentes est comprise entre 4 et 20 mm.

13. Gouttière pour pied et cheville selon l'une quelconque des revendications précédentes, **caractérisée en ce que** la distance comprise entre les fentes adjacentes est à peu près la même pour des fentes adjacentes différentes.

14. Gouttière pour pied et cheville selon l'une quelconque des revendications précédentes, **caractérisée en ce que** la largeur des fentes est comprise entre 0,3 et 1,2 mm.

15. Gouttière pour pied et cheville selon l'une quelconque des revendications précédentes, **caractérisée en ce que** le corps comporte, aux extrémités d'un certain nombre de fentes, un trou débouchant.

16. Gouttière pour pied et cheville selon la revendication 15, **caractérisée en ce qu'**un certain nombre de trous débouchants ont un diamètre compris entre 1,5 et 8 mm.

17. Gouttière pour pied et cheville selon l'une quelconque des revendications précédentes, **caractérisée en ce que** les fentes sont disposées sur un trajet pratiquement vertical au niveau de la cheville sur une distance d'environ 4 à 10 cm.

18. Gouttière pour pied et cheville selon l'une quelconque des revendications précédentes, **caractérisée en ce que** le corps possède une ouverture disposée au-dessus des fentes.

19. Gouttière pour pied et cheville selon l'une quelconque des revendications précédentes, **caractérisée en ce qu'**une première sangle de fixation est disposée à une certaine hauteur au-dessus des fentes.

20. Gouttière pour pied et cheville selon la revendication 18, **caractérisée en ce qu'**une seconde sangle de fixation se trouve au niveau du cou-de-pied du pied.

21. Gouttière pour pied et cheville selon l'une quelconque des revendications précédentes, **caractérisée en ce que** le corps en forme de botte est fabriqué en un matériau rigide et élastique.
